# EUROPEAN PATENT SPECIFICATION

(11) **EP 2 493 446 B1**
(45) Date of publication and mention of the grant of the patent: **21.03.2018**
(21) Application number: 09741557.4
(22) Date of filing: 26.10.2009
(51) Int. Cl.: A61K 8/36, A61K 8/44, A61K 8/49, A61Q 11/00, A61K 8/22, A61K 8/27

(54) **ORAL COMPOSITION FOR TREATING ORAL MALODOR**
ORALE ZUSAMMENSETZUNG GEGEN MUNDGERUCH
COMPOSITION ORALE CONTRE LA MAUVAISE HALEINE

(43) Date of publication of application: 05.09.2012
(73) Proprietor: Colgate-Palmolive Company, New York, NY 10022 (US)
(72) Inventor: PILCH, Shira, Highland Park NJ 08904 (US); MASTERS, James, Ringoes, NJ 08551 (US); WON, Betty, New Brunswick, NJ 08901 (US)
(74) Representative: Wibbelmann, Jobst
(86) International application number: PCT/US2009/062017
(87) International publication number: WO 2011/053273

(56) References cited:
- WO-A1-95/07682
- WO-A1-96/05803
- WO-A1-99/17735
- WO-A1-03/075865
- WO-A1-2008/006725
- CN-A- 1 313 120
- JP-A- 2007 254 315

## Description

### BACKGROUND OF THE INVENTION

The present invention relates to oral care compositions and the use of such oral care compositions for treating oral malodor. The present invention also relates to a method for treatment or prevention of oral malodor.

Halitosis, the technical term for bad breath, or oral malodor (sometimes referred to as Fetor ex Ore), is an undesirable condition. As a matter of fact, everyone, excluding the very young, occasionally has bad breath, with approximately 25% suffering on a regular basis and the problem tends to get worse and more frequent as one gets older. The problem seems to be evenly split between men and women. Bad breath results when proteins from the food we eat and saliva debris are broken down by bacteria. Even the cleanest mouth hosts bacteria that have the potential to decompose these protein-containing particles left in the mouth. The tongue, with its fissures and large, bumpy surface area, retains considerable quantities of food and debris that support and protect a large bacterial population. Under low oxygen conditions, this bacterial population forms foul smelling products, called volatile sulfur compounds (VSC)-such as hydrogen sulfide ("rotten eggs") and methyl mercaptans ("skunk smell") and other odorous and bad tasting compounds. Up to 80-90% of bad breath that originates in the mouth is believed to be caused by this mechanism.

Current instrument analyses of oral malodor are aimed at measuring VSC levels. There is a poor correlation, however, between organoleptic assessment, which is the standard oral malodor measurement, and measured VSC levels. This has led to a growing interest in non-VSC malodor components. Reduction of the non-VSC malodor components offers a new approach for development of novel oral malodor treatment technologies.

A type of non-VSC malodor components are short-chain carboxylic acids, such as butyric acid. Another type of non-VSC malodor components are amines, such as putrescine, cadaverine, and indole. Indole is an amine present in oral malodor that has a lower odor threshold than hydrogen sulfide. Therefore, molecule for molecule, indole is more odorous than hydrogen sulfide. Indole is formed from the degradation of tryptophan in the presence of tryptophanase.

A number of documents disclose various oral care compositions for treating oral malodour. For example, US 5738840 discloses an oral care composition for reduction of oral malodor, in which the composition contains molecular chlorine dioxide at a concentration of about 1 ppm to about 200 ppm and a pH in the range of about 5.0 to about 7.5. US 6159447 discloses compositions for controlling bacterial growth and colonization, in which the compositions contain an enzyme and an anchor molecule coupled to the enzyme, where the anchor molecule is capable of attaching to a substrate proximal to bacterial colony.

US 6723305 discloses a mouthrinse containing cetylpyridinium chloride and zinc ions for antibacterial effect that removes odor creating bacteria from the oral cavity. US 7250162 discloses a lactic acid bacteria strain for treatment of oral malodor. US 7297327 discloses an oral odor control agent admixture containing thymol, anise, fennel, basil, and juniperberry essential oils for control of garlic odor. US 7402416 discloses an oral care composition for reduction of plaque and oral malodor, which contains a non-ionic antibacterial compound and enzyme containing dentifrice.

US 2003/0158111 discloses oral care compositions containing metal-binding peptides, peptide derivatives, and peptide dimers for reduction of inflammation of tissues of the mouth and for reduction of damage done by reactive oxygen species to the tissues of the mouth. US 2006/0008425 discloses an oral care composition containing an enzyme and a cyclodextrin. US 2008/0152600 discloses peptides that bind oral surfaces such as teeth and gums. The disclosed peptides are used for delivery of oral care benefit agents to oral cavity surfaces. US 2008/0254079 discloses various compositions, including oral care compositions, containing a lysozyme, a polysaccharide, and, optionally, serine protease.

WO-A-2008/006725 discloses a toothpaste composition. WO-A-96/05803 discloses breath malodour reduction. CN-A-1313120 discloses a face-beautifying buccal lozenge and its preparation. JP-A-2007,254315 discloses a rock salt-containing sanitary composition for the oral cavity. WO-A-99/17735 discloses a mouth hygienic composition for the treatment of halitosis.

### SUMMARY OF THE INVENTION

There is a need in the art to provide an improved oral composition capable of treatment or prevention of oral malodor. Furthermore, there is a need in the art to provide oral care composition capable of treatment of non-VSC malodor.

The present invention provides an oral care composition according to claim 1. The present invention also provides a method as defined in the claims for treatment or prevention of oral malodour caused by hydrogen sulfide and indole. Preferred features are defined in the dependent claims.

### DETAILED DESCRIPTION

The following definitions and non-limiting guidelines must be considered in reviewing the description of this invention set forth herein. The headings (such as "Background" and "Brief Summary,") and sub-headings (such as "Compositions" and "Methods") used herein are intended only for general organization of topics within the disclosure of the invention, and are not intended to limit the disclosure of the invention or any aspect thereof. In particular, subject matter disclosed in the "Background" may include aspects of technology within the scope of the invention, and may not constitute a recitation of prior art. Subject matter disclosed in the "Brief Summary" is not an exhaustive or complete disclosure of the entire scope of the invention or any embodiments thereof. Classification or discussion of a material within a section of this specification as having a particular utility (e.g., as being an "active" or a "carrier" ingredient) is made for convenience, and no inference should be drawn that the material must necessarily or solely function in accordance with its classification herein when it is used in any given composition.

The citation of references herein does not constitute an admission that those references are prior art or have any relevance to the patentability of the invention disclosed herein. Any discussion of the content of references cited in the Background is intended merely to provide a general summary of assertions made by the authors of the references, and does not constitute an admission as to the accuracy of the content of such references.

Moreover, recitation of multiple embodiments having stated features is not intended to exclude other embodiments having additional features, or other embodiments incorporating different combinations the stated of features. Examples are provided for illustrative purposes of how to make and use the compositions and methods of this invention and, unless explicitly stated otherwise, are not intended to be a representation that given embodiments of this invention have, or have not, been made or tested.

As used herein, the words "preferred" and "preferably" refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention. In addition, the compositions and the methods may comprise, consist essentially of, or consist of the elements described therein.

As used throughout, ranges are used as a shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material. The recitation of a specific value herein is intended to denote that value, plus or minus a degree of variability to account for errors in measurements. For example, an amount of 10% may include 9.5% or 10.5%, given the degree of error in measurement that will be appreciated and understood by those having ordinary skill in the art.

The term "serine" used in the context of the present invention means L-Serine amino acid, also known as (2*S*)-2-amino-3-hydroxypropanoic acid. The term "zinc salt" used in the context of the present invention means Zn²⁺ -containing compounds, such as, for example, zinc lactate, zinc citrate, and zinc chloride. The term "tin salt" used in the context of the present invention means Sn²⁺ -containing compounds, such as, for example, SnCl₂ , SnF₂, and Sn pyrophosphate.

As used herein, terms "treatment" or "treating" are intended to include prophylaxis. The terms include amelioration, prevention and relief from the symptoms and/or effects associated with oral malodor. The terms "preventing" or "prevention" refer to administering the composition of the invention beforehand to forestall or obtund oral malodor. Persons of ordinary skill in the art of compositions for the treatment of oral malodor (to which the present method claims are directed) recognize that the term "prevent" is not an absolute term. Rather, the term is understood to refer to the prophylactic administration of a composition to diminish the likelihood or seriousness of a condition, and this is the sense intended.

Serine is a naturally-occurring amino acid having the formula HO2CCH(NH)2CH2OH. Due to its structural similarity to cysteine (HO2CCH(NH)2CH2SH), serine is believed to be a substrate competitor of cysteine for cysteine desulflrydrase. (Delwiche, J. Bacteriol., 62, 717-722 (1951)). Cysteine desulfhydrase is believed to be responsible for increasing levels of hydrogen sulfide, a VSC. More specifically, cysteine desulfhydrase catalyzes the degradation of cysteine to hydrogen sulfide. Serine also is believed to be capable of down regulating the enzymatic degradation of tryptophan by reacting with indole to form tryptophan in the presence of tryptophan synthase (Lane and Kirschner, Eur. J. Biochem., 129, 571-582 (1983)).

The present inventors' research has now demonstrated that serine produces a significant reduction in VSC when incubated with whole saliva and cysteine. In addition, the inventors have developed a sample preparation method using Gas Chromatography ("GC") to quantify the effect of serine on indole formation. As a result, it has now been discovered that as the level of serine added to whole saliva is increased, the indole concentration is decreased. Thus, it has now been discovered that serine exhibits a dual function toward malodor control with its ability to control both VSC and indole production.

### Composition

In an embodiment, the present invention provides an oral care composition as defined in the claims, particularly comprising serine, in which serine is present in an amount effective to reduce oral malodor caused by hydrogen sulfide. In one such embodiment, serine of the oral care composition is present in an amount sufficient to lower degradation of cysteine to hydrogen sulfide by cysteine desulfhydrase in an oral cavity. This results in lowering of the amount of hydrogen sulfide, a VSC gas, in an oral cavity. Thus, lowering of the amount of hydrogen sulfide results in treatment or prevention of oral malodor.

In an embodiment, the present invention provides an oral care composition comprising serine, in which serine is present in an amount effective to reduce oral malodor caused by indole. In one such embodiment, serine of the oral care composition is present in an amount sufficient to react with indole in the presence of tryptophan synthase to form tryptophan in an oral cavity. This results in lowering of the amount of indole in an oral cavity. Indole is believed to be one of the causes of oral malodor. Thus, lowering of the amount of indole results in treatment or prevention of oral malodor.

In an embodiment, the present invention provides an oral care composition as defined in the claims, particularly comprising serine, wherein serine is present in an amount effective to reduce oral malodor caused by hydrogen sulfide and indole. In one such embodiment, serine is present in an amount sufficient to lower degradation of cysteine to hydrogen sulfide by cysteine desulfhydrase and in an amount sufficient to react with indole in the presence of tryptophan synthase to form tryptophan in an oral cavity.

In an embodiment, the present invention provides an oral care composition as defined in the claims, particularly comprising serine, in which serine is present in an amount effective to reduce oral malodor caused by at least one component selected from the group consisting of hydrogen sulfide, indole, and mixtures thereof.

In one embodiment, serine is present in an amount sufficient to lower degradation of cysteine to hydrogen sulfide by cysteine desulfhydrase in an oral cavity. In another embodiment, serine is present in an amount sufficient to react with indole in the presence of tryptophan synthase to form tryptophan in an oral cavity. In yet another embodiment, serine is present in an amount sufficient to lower degradation of cysteine to hydrogen sulfide by cysteine desulfhydrase and in an amount sufficient to react with indole in the presence of tryptophan synthase to form tryptophan in an oral cavity.

Serine is present in the compositions of the invention at a concentration of 0.01 to 10% w/w, preferably at a concentration of 0.1 to 5% w/w, most preferably at a concentration of 0.4 - 1% w/w.

In an embodiment, the compositions of the invention further comprise one or more agents selected from oxidizing agents, and antimicrobial agents. The oxidizing agents, metal-chelating agents, and antimicrobial agents are not limited for use in the various embodiments, and any oxidizing agents, metal-chelating agents, and antimicrobial agents can be used in the embodiments. Preferred oxidizing agents may be selected from H₂O₂ and ClO₂. Preferred metal-chelating agents may be selected from **Zn**^{**2**+} and Sn²⁺ - containing compounds. The antimicrobial agents may be selected from triclosan, cetylpyridinium chloride, chlorhexidine, botanical extracts, and one or more essential oils. Botanical extracts may be selected from any disclosed in United States Patent Application Publication No. 2009/0087501. Addition of oxidizing agents, metal-chelating agents, and/or antimicrobial agents can further enhance the efficacy of the compositions of the invention against oral malodor.

The present inventors have discovered that the efficacy of the compositions of the present invention may be increased when the compositions contain a metal-chelating agent which is a zinc species as defined in the claims in addition to serine.

As zinc salts are known to be VSC inhibitors, an additive effect may result from use of serine together with a zinc salt.

In an embodiment, zinc lactate is present in the compositions of the invention at a concentration of 0.01 to 3% w/w, preferably at a concentration of 0.1 to 0.5% w/w. Other salts of zinc may be used besides zinc lactate, such as zinc citrate or zinc chloride. For any salt of zinc used, the corresponding zinc ion is present at a concentration of about 0.003 to about 1% w/w, preferably, at a concentration of about 0.03 to about 0.2 % w/w.

In one embodiment, the compositions of the invention further comprise one or more agents selected from anti-plaque agents, whitening agents, sweetening agents, cleaning agents and flavoring agents. In another embodiment, the compositions of the invention comprise an orally acceptable carrier for a toothpaste, a dental cream, a mouthwash, a chewing gum or a denture adhesive.

### Methods Of Use

In an embodiment, the present invention provides a method of treatment or prevention of oral malodor, comprising applying to an oral cavity an oral care composition comprising serine and a zinc species as defined in the claims, in which serine is present in an amount effective to reduce aral malodor caused by hydrogen sulfide. In an embodiment, serine is present in an amount sufficient to lower degradation of cysteine to hydrogen sulfide by cysteine desulfhydrase in an oral cavity, resulting in a reduction in the amount of hydrogen sulfide in an oral cavity. Accordingly, in one embodiment, the present invention is directed to a method of lowering of the amount of hydrogen sulfide in an oral cavity.

In an embodiment, the present invention provides a method of treatment or prevention of oral malodor, comprising applying to an oral cavity an oral care composition comprising serine, wherein serine is present in an amount effective to reduce oral malodor caused by indole. In an embodiment, serine is present in an amount sufficient to react with indole in the presence of tryptophan synthase to form tryptophan in an oral cavity, resulting in a reduction in the amount of indole in an oral cavity. Accordingly, in one embodiment, the present invention is directed to a method of lowering of the amount of indole in an oral cavity.

In an embodiment, the present invention provides a method of treatment or prevention of oral malodor, comprising applying to an oral cavity an oral care composition comprising serine, wherein serine is present in an amount effective to reduce oral malodor caused by hydrogen sulfide and indole. In an embodiment, serine is present in an amount sufficient to lower degradation of cysteine to hydrogen sulfide by cysteine desulfhydrase and in an amount sufficient to react with indole in the presence of tryptophan synthase to form tryptophan in an oral cavity. Accordingly, in one embodiment, the present invention is directed to a method of lowering of the amount of hydrogen sulfide and indole in an oral cavity.

In an embodiment, the present invention provides a method of treatment or prevention of oral malodor, comprising applying to an oral cavity an oral care composition comprising serine, wherein serine and a zinc species as defined in the claims is present in an amount effective to reduce oral malodor caused by at least one component selected from the group consisting of hydrogen sulfide, indole, and mixtures thereof.

In the methods of the present invention, serine is present in the composition at a concentration of 0.01 to 10% w/w, preferably, at a concentration of 0.1 to 5% w/w. In one embodiment, serine may be present at a concentration of 0.4 to 1% w/w.

In an embodiment, the composition to be used in the methods of the present invention further comprises one or more agents selected from oxidizing agents, and antimicrobial agents. The oxidizing agents, metal-chelating agents, and antimicrobial agents are not limited for use in the various embodiments, and any oxidizing agents, metal-chelating agents, and antimicrobial agents can be used in the embodiments. Preferred oxidizing agents may be selected from H₂O₂ and ClO₂.

The antimicrobial agents may be selected from triclosan, cetylpyridinium chloride, chlorhexidine, botanical extracts, and one or more essential oils.

In one embodiment, the composition to be used in the methods of the present invention further comprises one or more agents selected from anti-plaque agents, whitening agents, sweetening agents, cleaning agents and flavoring agents. In another embodiment, the composition to be used in the methods of the present invention comprises an orally acceptable carrier for a toothpaste, a dental cream, a mouthwash, a chewing gum or a denture adhesive.

Various embodiments now will be described with reference to the following non-limiting examples.

### SPECIFIC EMBODIMENTS OF THE INVENTION

The invention is further described in the following examples. The examples are merely illustrative and do not in any way limit the scope of the invention as described and claimed.

### Example 1: Reduction Of VSC With Serine (not claimed)

The effect of serine and other amino acids on VSC was tested. Whole saliva was incubated overnight with cysteine and an additional amino acid selected from serine, proline, tyrosine, glycine, aspargine, glutamic acid, and histidine. In this experiment, 10% of 0.1% amino acid solution was added to 50% whole saliva. GC measurements were made for VSC. Serine was found to show the greatest percent reduction of VSC relative to a sample containing saliva and cysteine alone. The results are presented in Table 1.

**Table 1.**

| **Amino Acid** | **VSC Reduction (%)** |
|---|---|
| Serine | 36.56 |
| Proline | 6.99 |
| Tyrosine | 0.06 |
| Glycine | 14 |
| Asparagine | -2.14 |
| Glutamic acid | -5.2 |
| Histidine | 1.36 |

The results show that serine was able to reduce VSC in saliva.

### Example 2: Reduction Of Indole With Serine (not claimed)

The effect of serine on indole was tested. Whole saliva with tryptophan (0.14%) and various concentrations of serine was incubated overnight at 37 °C. After 24 h, the samples were allowed to cool to 25 °C. The samples were extracted with hexanes and the organic layer was used for GC quantification. Table 2 demonstrates the effect of serine concentration on the formation of indole.

**Table 2.**

| **Serine (%)** | **Area Under the Curve (AUC)** |
|---|---|
| 0 | 642403 |
| 0.21 | 584720 |
| 0.42 | 532053 |
| 0.84 | 542136 |

The results show that serine was able to reduce amount of indole in saliva.

### Example 3: Reduction Of VSC With Zinc Lactate And Serine

The effect of zinc lactate and serine on VSC was tested. Whole saliva with tryptophan (0.14%), zinc lactate (0.16%), and when necessary, serine (0.42%), was incubated overnight at 37 °C. After 24 h, the samples were allowed to cool to 25 °C. The samples were then quantified for headspace VSC using a GC. Table 3 demonstrates the effect of zinc lactate with and without serine on VSC production.

**Table 3.**

| **Sample** | **VSC (ppb)** |
|---|---|
| Zinc Lactate | 109.25 |
| Zinc Lactate and Serine | 82.26 |

The results show that serine enhances the effect of zinc lactate and reduces VSC production.

### Example 4: Reduction Of Indole With Zinc Lactate And Serine

The effect of zinc lactate and serine on indole was tested. Whole saliva with tryptophan (0.14%), various concentrations of zinc lactate, and when necessary, serine (0.42%), was incubated overnight at 37 °C. After 24 h, the samples were allowed to cool to 25 °C. The samples were extracted with hexanes (2 mL) and the organic layer (1 mL) was used for GC quantification. Table 4 demonstrates effect of zinc lactate and zinc lactate with serine on indole production.

**Table 4.**

| **Sample** | **Indole (ppm)** |
|---|---|
| 0.23% Zinc Lactate | 4.71 |
| 0.23% Zinc Lactate and Serine | 4.03 |
| 0.16% Zinc Lactate | 5.09 |
| 0.16% Zinc Lactate and Serine | 4.09 |
| 0.09% Zinc Lactate | 9.99 |
| 0.09% Zinc Lactate and Serine | 8.70 |

The results show that serine enhances the effect of zinc lactate and reduces indole production,

### Example 5: Oral Dentifrice Composition With Serine (not claimed)

Table 5 illustrates an example of an oral dentifrice composition containing serine.

**Table 5.**

| **Ingredient** | **% wt/wt** |
|---|---|
| Sorbitol | 68 |
| Water | 8.95 |
| Polyethylene glycol 600 | 3 |
| Silica | 16 |
| Sodium Lauryl Sulfate | 1.5 |
| NaF | 0.3 |
| Flavor | 1 |
| Sodium Carboxymethyl Cellulose | 0.6 |
| Sodium Saccharin | 0.35 |
| Serine | 0.3 |
| Cocamidopropyl Betaine | 0.375 |

### Example 6: Oral Mouthwash Composition With Serine (not claimed)

Table 6 illustrates an example of an oral mouthwash composition containing serine.

**Table 6.**

| **Ingredient** | **% wt/wt** |
|---|---|
| Glycerin | 7.5 |
| Propylene Glycol | 7 |
| Water | 77.89 |
| Poloxomer | 0.4 |
| Flavor | 0.1 |
| Cetylpyridinium Chloride | 0.05 |
| Parabens | 0.04 |
| Sodium Saccharin | 0.02 |
| Sodium Fluoride | 0.05 |
| Sorbitol | 6 |
| L-Serine | 1 |

### Example 7: Oral Dentifrice Composition Containing Serine And Zinc Lactate

Table 7 illustrates an example of an oral dentifrice composition containing serine and zinc lactate.

**Table 7.**

| **Ingredient** | **% wt/wt** |
|---|---|
| Sorbitol | 68 |
| Water | 6.805 |
| Polyethylene glycol 600 | 3 |
| Silica | 16 |
| Sodium Lauryl Sulfate | 2.2 |
| NaF | 0.32 |
| Flavor | 1.15 |
| Sodium Carboxymethyl Cellulose | 0.6 |
| Zinc Lactate | 0.2 |
| Sodium Saccharin | 0.35 |
| L-Serine | 1 |
| Cocamidopropyl Betaine | 0.375 |

### Example 8: Oral Mouthwash Composition Containing Serine And Zinc Lactate

Table 8 illustrates an example of an oral mouthwash composition containing serine and zinc lactate.

**Table 8.**

| **Ingredient** | **% wt/wt** |
|---|---|
| Glycerin | 7.5 |
| Propylene Glycol | 7 |
| Water | 78.05 |
| Poloxamer | 0.4 |
| Flavor | 0.165 |
| Cetylpyridinium Chloride | 0.075 |
| Parabens | 0.04 |
| Sodium Saccharin | 0.02 |
| Sodium Fluoride | 0.05 |
| Sorbitol | 5.5 |
| Zinc Lactate | 0.2 |
| L-Serine | 1 |

The examples and other embodiments described herein are exemplary and not intended to be limiting in describing the full scope of compositions and methods of this invention. Equivalent changes, modifications and variations of specific embodiments, materials, compositions and methods may be made within the scope of the present invention, with substantially similar results.

## Claims

1. An oral care composition comprising L-serine amino acid, wherein L-serine amino acid is present in an amount effective to reduce oral malodor caused by hydrogen sulfide and indole, L-serine amino acid being present in the composition at a concentration of 0.01 to 10% w/w, and a metal-chelating agent, which is zinc lactate.

2. The oral care composition of claim 1, wherein L-serine amino acid is present in the composition at a concentration of 0.1 to 5% w/w, optionally at a concentration of 0.4 to 1 % w/w.

3. The oral care composition of claim 1, wherein the composition further comprises one or more agents selected from oxidizing agents and antimicrobial agents.

4. The oral care composition of claim 3, wherein one or more oxidizing agents are selected from H₂O₂ any ClO₂.

5. The oral care composition of claim 1, wherein the zinc lactate is present in the composition at a concentration of 0.1 to 0.5 % w/w.

6. The oral care composition of claim 3, wherein one or more antimicrobial agents are selected from a group consisting of triclosan, cetylpyridinium chloride, chlorhexidine, botanical extracts, and one or more essential oils.

7. The oral care composition of claim 1, further comprising one or more agents selected from anti-plaque agents, whitening agents, sweetening agents, cleaning agents and flavoring agents.

8. The oral care composition of claim 1, further comprising an orally acceptable carrier for a toothpaste, a dental cream, a mouthwash, a chewing gum or a denture adhesive.

9. A method of treatment or prevention of oral malodor caused by hydrogen sulfide and indole, comprising applying to an oral cavity an oral care composition comprising L-serine amino acid, wherein L-serine amino acid is present in an amount effective to reduce oral matador caused by hydrogen sulfide and indole, L-serine amino acid being present in the composition at a concentration of 0.01 to 10% w/w, and a metal-chelating agent, which is zinc lactate.

10. The method of claim 9, wherein L-serine amino acid is present in the composition at a concentration of 0.1 to 5% w/w, optionally at a concentration of 0.4 to 1% w/w.

11. The method of claim 9, wherein the composition further comprises one or more agents selected from oxidizing agents and antimicrobial agents.

12. The method of claim 11, wherein one or more oxidizing agents are selected from a group consisting of H₂O₂ and ClO₂.

13. The method of claim 9, wherein the zinc lactate is present in the composition at a concentration of 0.1 to 0.5 % w/w.

14. The method of claim 11, wherein one or more antimicrobial agents are selected from a group consisting of triclosan, cetylpyridinium chloride, chlorhexidine, botanical extracts, and one or more essential oils.

15. The method of claim 9, wherein the composition further comprises one or more agents selected from anti-plaque agents, whitening agents, sweetening agents, cleaning agents and flavoring agents.

16. The method of claim 9, wherein the composition comprises an orally acceptable carrier for a toothpaste, a dental cream, a mouthwash, a chewing gum or a denture adhesive.

## Patentansprüche

1. Mundpflegezusammensetzung, die L-Serin-Aminosäure umfasst, wobei L-Serin-Aminosäure in einer Menge vorliegt, die wirksam ist, durch Schwefelwasserstoff und Indol verursachten Mundgeruch zu verringern, wobei L-Serin-Aminosäure in der Zusammensetzung in einer Konzentration von 0,01 bis 10% Gew./Gew. vorliegt, und einen Metallkomplexbildner, der Zinklactat ist.

2. Mundpflegezusammensetzung nach Anspruch 1, wobei L-Serin-Aminosäure in der Zusammensetzung in einer Konzentration von 0,1 bis 5% Gew./Gew., gegebenenfalls in einer Konzentration von 0,4 bis 1% Gew./Gew. vorliegt.

3. Mundpflegezusammensetzung nach Anspruch 1, wobei die Zusammensetzung weiterhin ein oder mehrere Mittel umfasst, ausgewählt aus Oxidationsmitteln und antimikrobiellen Mitteln.

4. Mundpflegezusammensetzung nach Anspruch 3, wobei ein oder mehrere Oxidationsmittel aus H₂O₂ und ClO₂ ausgewählt sind.

5. Mundpflegezusammensetzung nach Anspruch 1, wobei das Zinklactat in der Zusammensetzung in einer Konzentration von 0,1 bis 0,5% Gew./Gew. vorliegt.

6. Mundpflegezusammensetzung nach Anspruch 3, wobei das eine oder die mehreren antimikrobiellen Mittel aus einer Gruppe bestehend aus Triclosan, Cetylpyridiniumchlorid, Chlorhexidin, botanischen Extrakten und einem oder mehreren ätherischen Ölen ausgewählt ist/sind.

7. Mundpflegezusammensetzung nach Anspruch 1, die weiterhin ein oder mehrere Mittel umfasst, die aus Anti-Plaque-Mitteln, Aufhellungsmitteln, Süßungsmitteln, Reinigungsmitteln und Aromastoffen ausgewählt sind.

8. Mundpflegezusammensetzung nach Anspruch 1, die weiterhin einen oral verträglichen Träger für eine Zahnpasta, eine Zahncreme, ein Mundwasser, einen Kaugummi oder ein Zahnprothesenhaftmittel umfasst.

9. Verfahren zur Behandlung oder Vorbeugung von durch Schwefelwasserstoff und Indol verursachtem Mundgeruch, das Anwenden einer L-Serin-Aminosäure umfassenden Mundpflegezusammensetzung auf eine Mundhöhle umfasst, wobei L-Serin-Aminosäure in einer Menge vorliegt, die wirksam ist, durch Schwefelwasserstoff und Indol verursachten Mundgeruch zu verringern, wobei L-Serin-Aminosäure in der Zusammensetzung in einer Konzentration von 0,01 bis 10% Gew./Gew. vorliegt, und einen Metallkomplexbildner, der Zinklactat ist.

10. Verfahren nach Anspruch 9, wobei L-Serin-Aminosäure in der Zusammensetzung in einer Konzentration von 0,1 bis 5% Gew./Gew., gegebenenfalls in einer Konzentration von 0,4 bis 1% Gew./Gew. vorliegt.

11. Verfahren nach Anspruch 9, wobei die Zusammensetzung weiterhin ein oder mehrere Mittel umfasst, ausgewählt aus Oxidationsmitteln und antimikrobiellen Mitteln.

12. Verfahren nach Anspruch 11, wobei ein oder mehrere Oxidationsmittel aus einer Gruppe bestehend aus H₂O₂ und ClO₂ ausgewählt sind.

13. Verfahren nach Anspruch 9, wobei das Zinklactat in der Zusammensetzung in einer Konzentration von 0,1 bis 0,5% Gew./Gew. vorliegt.

14. Verfahren nach Anspruch 11, wobei das eine oder die mehreren antimikrobiellen Mittel aus einer Gruppe bestehend aus Triclosan, Cetylpyridiniumchlorid, Chlorhexidin, botanischen Extrakten und einem oder mehreren ätherischen Ölen ausgewählt ist/sind.

15. Verfahren nach Anspruch 9, wobei die Zusammensetzung weiterhin ein oder mehrere Mittel umfasst, die aus Anti-Plaque-Mitteln, Aufhellungsmitteln, Süßungsmitteln, Reinigungsmitteln und Aromastoffen ausgewählt sind.

16. Verfahren nach Anspruch 9, wobei die Zusammensetzung weiterhin einen oral verträglichen Träger für eine Zahnpasta, eine Zahncreme, ein Mundwasser, einen Kaugummi oder ein Zahnprothesenhaftmittel umfasst.

## Revendications

1. Composition de soin buccal comprenant un acide aminé L-sérine, dans laquelle l'acide aminé L-sérine est présent en une quantité efficace pour réduire la mauvaise haleine causée par le sulfure d'hydrogène et l'indole, l'acide aminé L-sérine étant présent dans la composition à une concentration de 0,01 à 10 % en poids, et un agent chélatant les métaux, qui est le lactate de zinc.

2. Composition de soin buccal selon la revendication 1, dans laquelle l'acide aminé L-sérine est présent dans la composition à une concentration de 0,1 à 5 % en poids/poids, éventuellement à une concentration de 0,4 à 1 % en poids/poids.

3. Composition de soin buccal selon la revendication 1, dans laquelle la composition comprend en outre un ou plusieurs agents choisis parmi les agents oxydants et les agents antimicrobiens.

4. Composition de soin buccal selon la revendication 3, dans laquelle un ou plusieurs agents oxydants sont choisis parmi H₂O₂ et ClO₂.

5. Composition de soin buccal selon la revendication 1, dans laquelle le lactate de zinc est présent dans la composition à une concentration de 0,1 à 0,5 % en poids/poids.

6. Composition de soin buccal selon la revendication 3, dans laquelle un ou plusieurs agents antimicrobiens sont choisis dans le groupe constitué par le triclosan, le chlorure de cétylpyridinium, la chlorhexidine, des extraits botaniques et une ou plusieurs huiles essentielles.

7. Composition de soin buccal selon la revendication 1, comprenant en outre un ou plusieurs agents choisis parmi les agents anti-plaque, les agents de blanchiment, les agents édulcorants, les agents de nettoyage et les agents aromatisants.

8. Composition de soin buccal selon la revendication 1, comprenant en outre un support acceptable par voie orale pour une pâte dentifrice, une crème dentaire, un bain de bouche, un chewing-gum ou un adhésif pour prothèse dentaire.

9. Procédé de traitement ou de prévention de la mauvaise haleine causée par le sulfure d'hydrogène et l'indole, comprenant l'application à une cavité buccale d'une composition de soin buccal comprenant un acide aminé L-sérine, dans laquelle l'acide aminé L-sérine est présent en une quantité efficace pour réduire la mauvaise haleine causée par le sulfure d'hydrogène et l'indole, l'acide aminé L-sérine étant présent dans la composition à une concentration de 0,01 à 10 % en poids/poids, et un agent chélatant les métaux, qui est le lactate de zinc.

10. Procédé selon la revendication 9, dans lequel l'acide aminé L-sérine est présent dans la composition à une concentration de 0,1 à 5 % en poids/poids, éventuellement à une concentration de 0,4 à 1 % en poids/poids.

11. Procédé selon la revendication 9, dans lequel la composition comprend en outre un ou plusieurs agents choisis parmi les agents oxydants et les agents antimicrobiens.

12. Procédé selon la revendication 11, dans lequel un ou plusieurs agents oxydants sont choisis dans un groupe constitué par H₂O₂ et ClO₂.

13. Procédé selon la revendication 9, dans lequel le lactate de zinc est présent dans la composition à une concentration de 0,1 à 0,5 % en poids/poids.

14. Procédé selon la revendication 11, dans lequel un ou plusieurs agents antimicrobiens sont choisis dans un groupe comprenant le triclosan, le chlorure de cétylpyridinium, la chlorhexidine, des extraits botaniques et une ou plusieurs huiles essentielles.

15. Procédé selon la revendication 9, dans lequel la composition comprend en outre un ou plusieurs agents choisis parmi les agents anti-plaque, les agents de blanchiment, les agents édulcorants, les agents de nettoyage et les agents aromatisants.

16. Procédé selon la revendication 9, dans lequel la composition comprend un support acceptable par voie orale pour un dentifrice, une crème dentaire, un bain de bouche, un chewing-gum ou un adhésif pour prothèse dentaire.
